# EUROPÄISCHE PATENTSCHRIFT

(11) **EP 2 368 972 B1**
(45) Veröffentlichungstag und Bekanntmachung des Hinweises auf die Patenterteilung: **15.05.2013**
(21) Anmeldenummer: 10003051.9
(22) Anmeldetag: 23.03.2010
(51) Int. Cl.: C11D 1/825, C11D 17/00, A61K 8/06

(54) **Hautfreundliche Handgeschirrspülmittel**
Eudermic manual dishwashing composition
Produit de nettoyage manuel de vaisselle doux pour la peau

(43) Veröffentlichungstag der Anmeldung: 28.09.2011
(73) Patentinhaber: Cognis IP Management GmbH, 40589 Düsseldorf (DE)
(72) Erfinder: Hloucha, Matthias,, 50677 Köln (DE); Küsters, Esther, 40625 Düsseldorf, (DE); Menzer, Jasmin,, 40789 Monheim, (DE); Prinz, Daniela,, 41542 Dormagen, (DE); Holz, Martina,, 40724 Hilden, (DE); Albrs, Thomas, Dr.,, 40597 Düsseldorf, (DE)

(56) Entgegenhaltungen:
- EP-A2- 0 559 304
- EP-A2- 1 152 051
- US-B1- 6 821 939

## Beschreibung

Die vorliegende Anmeldung betrifft hautfreundliche Handgeschirrspülmittel, sowie ein Verfahren zur Herstellung derartiger Mittel.

Zur manuellen Reinigung von verschmutztem Geschirr werden üblicherweise konzentrierte üblicherweise wässerige Tensidlösungen verwendet, an die heutzutage eine ganze Reihe teils sehr unterschiedlicher Anforderungen gestellt werden. Die Mittel sollen
- einen möglichst hohen Aktivsubstanzgehalt aufweisen,
- dabei flüssig oder zumindest fliessfähig sein
- einen möglichst niedrige Kältetrtrübungspunkt besitzen,
- auch bei hoher Fettbelastung der Flotte noch einen kräftigen, beständigen Schaum entwickeln und gleichzeitig natürlich
- über ein hohes Tellerspülvermögen verfügen,
- auch in konzentrierter Form dermatologisch unbedenklich, d. h. nicht hautreizend sein.

Ein besonderes Augenmerk verdient der Punkt der dermatologischen Unbedenklichkeit, da hier die Verbraucherwartungen in den letzten Jahren gestiegen sind. Vor dem Hintergrund einer steigenden Anzahl von Konsumenten, die eine empfindliche Haut haben, werden Mittel die diesbezüglich vorteilhaften Eigenschaften aufweisen immer wichtiger.

Die WO 00/71658 schlägt vor, in Handgeschirrspülmittel ein hautfreundliches, polymeres Mittel als Schaumstabilisator einzuarbeiten, um auf diese Weise nicht Haut irritierende Reinigungsmittel herzustellen.

Die EP 0 410 567 A1 schlägt vor, Handgeschirrspülmittel durch Zugabe bestimmter hautfreundlicher Additive, ausgewählt aus der Gruppe der Kohlenwasserstoffe, Ester, Amine, Amide, quaternären Ammoniumverbindungen oder von Alkoholen hautüeundlicher zu machen. Eine ausgewählte Komponente sind Wachse, insbesondere Bienenwachs. Die Mittel gemäß der Lehre der Schrift werden hergestellt, indem man die festen Inhaltsstoffe aufschmilzt und dann bei Temperaturen von > 70 °C mit der wässerigen Phase verrührt.

Weitere Mittel sind aus EP 1 152 051 A2 und EP 559 304 sowie US 6 821 939 B1 bekannt.

Auch wenn im Markt somit heute eine Vielzahl von hautfreundlichen Handgeschirrspülmitteln vorhanden ist, besteht doch ein intensives Interesse sowohl von Seiten der Rohstofflieferanten und der Hersteller, Mittel aufzufinden, die die geforderte Aufgabenstellung besser erfüllen als die Produkte des Stands der Technik. Dies gilt insbesondere in Bezug auf ein möglichst einfaches Herstellverfahren für solche Mittel.

Es wurde nun gefunden, dass bestimmte Emulsionen die oben gestellte Aufgabe zu lösen vermögen.

Ein erster Gegenstand der vorliegenden Anmeldung ist gerichtet auf die Verwendung von Mikroemulsionen, enthaltend mindestens (a) ein Alkyl(oligo)glycosid, (b) ein von (a) unterschiedliches Co-Tensid, (c) eine nicht-wasserlösliche organische Ölkomponente, (d) ein Wachs und Wasser zur Herstellung von Reinigungsmitteln.

### Mikroemulsion

Mikroemulsionen sind an sich bekannt. Mikroemulsionen sind makroskopisch homogene, optisch transparente, niedrigviskose, thermodynamisch stabile Mischungen. Je nach verwendetem Tensidtyp zeigt die Mikroemulsion ein temperaturabhängiges Phasenverhalten. Vor allem manche nichtionischen Tenside, und hier vor allem Tenside, deren hydrophile Molekülteile aus Ethoxy- oder Propoxy-Gruppen aufgebaut sind, führen zu einem charakteristischen temperaturabhängigen Phasenverhalten bei Mikroemulsionen.

Voraussetzung für die Bildung von Mikroemulsionen ist eine extrem niedrige Grenzflächenspannung zwischen der wasser- und der ölreichen Phase. Diese kann bei den Mikroemulsionen Werte zwischen 10⁻¹ und 10⁻⁵ mNm⁻¹ annehmen.

Die mittleren Teilchengrößen der Mikroemulsionen liegen üblicherweise unter 100 nm, vorzugsweise zwischen 3 und 100 nm. Sie weisen eine hohe Transparenz auf und sind beim Zentrifugieren bei 2000 UPM für mindestens 30 Minuten gegenüber einer sichtbaren Phasenseparation stabil. Die Mikroemulsionen im Sinne der vorliegenden Lehre zeigen vorzugsweise eine mittlere Teilchengröße von weniger als 100 nm. Die Leitfähigkeit der erfindungsgemäßen Mikroemulsionen liegt vorzugsweise im Bereich von größer/gleich 500 µSi/cm und besonders bevorzugt bei größer/gleich 1000 µSi/cm. Ein bevorzugter Bereich liegt bei 800 bis 1500 µSi/cm. Die erfindungsgemäßen Mikroemulsionen sind vorzugsweise transparent, insbesondere zeigen sie eine Transparenz von gröβer/gleich 80 % bei 40 °C, wobei Transparenz-Werte von größer als 90 % bei 40 °C typisch sind. Bevorzugt sind solche Mikroemulsionen, die eine Transparenz, gemessen bei 40 °C von 95 bis 100 % aufweisen.

Die Herstellung der Mikroemulsionen erfolgt vorzugsweise einfach durch Vermischen der Ölphase mit den weiteren öllöslichen Inhaltsstoffen, Erwärmen der Ölphase über den Schmelzpunkt aller Bestandteile und anschließender Zugabe der wässrigen tensidhaltigen Phase. Die thermodynamisch stabile Mikroemulsion bildet sich dann spontan, ggf. muss noch etwas gerührt werden.

Die Mikroemulsionen gemäß der vorliegenden Lehre sind vorzugsweise vom Typ Ölin-Wasser (O/W). Die Mikroemulsionen enthalten als äußere Phase somit vorzugsweise Wasser, und weiterhin Alkyl(oligo)glycoside und eine davon unterschiedliches Co-Tensid, sowie eine Ölkomponente und ein Wachs. Die Mirkoemulsionen können aber auch als Wasser-in-Öl Emulsion vorliegen.

### Komponente (a)

Bevorzugt werden als Komponente (a) der vorliegenden Erfindung Mikroemulsionen auf Basis von Alkyl(oligo)glykosiden eingesetzt.

Alkyl- und Alkenyloligoglykoside stellen bekannte nichtionische Tenside dar, die der allgemeinen Formel (I) folgen,

R¹O-[G]ₚ (I)

in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl-und/oder Alkenyloligoglucoside. Die Indexzahl p in der allgemeinen Formel (I) gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Caprronalkohol, Caprylalkohol, Caprinalkohol und Undecylalkohol sowie deren technische Mischungen, wie sie beispielsweise bei der Hydrierung von technischen Fettsäuremethylestem oder im Verlauf der Hydrierung von Aldehyden aus der Roelen'schen Oxosynthese erhalten werden. Bevorzugt sind Alkyloligoglucoside der Kettenlänge C₈-C₁₀ (DP=1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylaikohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3. Die Mikroemulsionen im Sinne der vorliegenden Lehre enthalten die Komponente (a) vorzugsweise in Mengen von 1 bis 35 Gew.-%, bezogen auf das Gesamtgewicht der Mikroemulsion und vorzugsweise in Mengen von 2 bis 30 Gew.% und 4 bis 25 Gew.%.

### Komponente (b)

Als weitere zwingende Komponente enthalten die Mikroemulsionen ein Co-Tensid, das strukturell von der Komponente (a) verschieden sein muss. Geeignet sind hier insbesondere nichtionische Tenside, wie Fettalkoholalkoxylate und deren Derivat. Eine besonders bevorzugte Gruppe von Co-Tensiden im Sinne der vorliegenden Lehre sind aber Ester von Polyolen und vorzugsweise die Ester von Glycerin. Ganz besonders Bevorzugt sind Monoester des Glycerins, wobei als Säurekomponente Fettsäure mit 12 bis 22 C-Atomen besonders bevorzugt sind. Geeignet sind insbesondere Monocarbonsäuren, die sowohl linear als auch verzweigt sein können. Besonders bevorzugt sind lineare Fettsäuren, die mindestens eine C-C-Dappelbindung enthalten, wobei die Ölsäure hier als bevorzugtes Beispiel zu nennen ist. Somit ist eine mit Vorteil auszuwählende Komponente (b) das Glycerinmonoleat. Herstellbedingt, können bei handelüblichen Monoglyceriden auch untergeordnete Mengen (z.B. kleiner 5 oder kleiner 1 Gew.-%) an Di- und Triglyceriden, bzw. freiem Glycerin vorliegen, ohne dass dies die Wirkung des Monoglycerids beeinträchtigt.

Die Co-Tenside können auch als Mischung eingesetzt werden. Sie sind vorzugsweise in Mengen von 1 bis 20 Gew.-%, bezogen auf das Gesamtgewicht der Mikroemulsion und insbesondere in Mengen von 2 bis 15 Gew.-% und 4 bis 12 Gew.-% enthalten.

### Komponente (c)

Die Mikroemulsionen im sinne der vorliegenden Lehre enthalten als weiteren obligatorischen Bestandteil eine wasserunlösliche so genannte Ölkomponente, also eine nicht-wasserlösliche organische Phase, vorzugsweise in Mengen von 5 bis 30 Gew.-% bezogen auf das Gesamtgewicht der Mikroemulsion. Dabei sind besonders bevorzugte Ölphasen ausgewählt aus der Gruppe von Guerbetalkoholen auf Basis von Fettalkoholen mit 6 bis 18 C-Atomen, Estern linearer C₆-C₂₂-Fettsäuren mit linearen oder verzweigten C₆-C₂₂-Fettalkoholen bzw. Estern von verzweigten C₆-C₁₃-Carbonsäuren mit linearen oder verzweigten C₆-C₂₂-Fettalkoholen, Ester von linearen C₆-C₂₂-Fettsäuren mit verzweigten Alkoholen, Estern von C₆-C₂₂-Fettalkoholen und/oder Guerbetalkoholen mit aromatischen Carbonsäuren, Triglyceriden auf Basis C₆-C₁₀-Fottsäuren, flüssigen Mono-/Di-/Triglyceridmischungen auf Basis von C₆-C₁₈-Fettsäuren, Estern von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzlichen Öle, verzweigten primäre Alkoholen, substituierten Cyclohexanen, linearen und verzweigten C₆-C₂₂-Fettalkoholcarbonaten, Guerbetcarbonate auf Basis von Fettalkoholen mit 6 bis 18, vorzugsweise 8 bis 10 C Atomen, Estern der Benzoesäure mit linearen und/oder verzweigten C₆-C₂₂-Alkoholen, linearen oder verzweigten, symmetrischen oder unsymmetrischen Dialkylethern mit 6 bis 22 Kohlenstoffatomen pro Alkylgruppe, Ringöffnugsprodukten von epoxidierten Fettsäureestern mit Polyolen, Siliconölen und/oder aliphatischen bzw. naphthenischen Kohlenwasserstoffe, Dialkylcyclohexanen und/oder Silikonölen.

### Komponente (d)

Als weiteren Bestandteil enthalten die Mikroemulsionen Wachse. Diese können auch in Mischung mit den im vorherigen Abschnitt genannten Ölen vorliegen. Typische Beispiele für Wachse im Sinne der vorliegenden Lehre natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwaehs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Weiterhin fallen Ester langkettiger Fettsäuren mit langkettigen Fettalkoholen unter den Begriff der Wachse.

Hier sind insbesondere Ester aus Monocarbonsäuren mit mindestens 14 C-Atomen mit Fettalkoholen mit mindestens 14 C-Atomen gemeint.

### Weitere Inhaltsstoffe

Die Mikroemulsionen enthaltend zwingend Wasser, vorzugsweise in Mengen von 1 bis 90 Gew.-%, wobei Mengen von 10 bis 75 Gew.-% und insbesondere von 15 bis 65 Gew.-% bevorzugt sein können.

Daneben können die Mirkoemulsionen als zusätzliche Komponente (f) eine kationische Verbindung, insbesondere quaternäre Ammoniumverbindungen oder ein kationisches Polymer enthalten. Unter quaternären Ammoniumverbindungen werden hierbei insbesondere quaternierte Fettsäuretriethanolaminestersalze verstanden. Geeignet sind aber ebenso Alkylammoniumhalogenide.

Geeignete kationische Polymere sind beispielsweise kationische Cellulosederivate, wie z.B. eine quaternierte Hydroxyethylcellulose, kationische Stärke, Copolymere von Diallylammoniumsalzen und Acrylamiden, quaternierte Vinylpyrrolidon/Vinylimidazol-Polymere, wie z.B. Luviquat (BASF), Kondensationsprodukte von Polyglycolen und Aminen, quaternierte Kollagenpolypeptide, wie beispielsweise Lauryldimonium hydroxypropyl hydrolyzed collagen (Lamequat), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine), Copolymere der Acrylsäure mit Dimethyldiallyl-anunoniumchlorid (Merquat 550), Polyaminopolyamide, wie z.B. beschrieben in der FR-A 2252840 sowie deren vernetzte wasserlöslichen Polymere, kationische Chitinderivate, wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischen Guar-Gum,, quaternierte Ammoniumsalz-Polymere, wie z.B. Mirapol A-15, Mirapol AD-1, Mirapol AZ-1 der Firma Miranol.

Weitere bevorzugten kationischen Polymeren sind gewählt aus der Gruppe der Homo-oder Copolymere von Ester- oder Amidderivaten der Acryl- oder Methacrylsäure (z.B. INCI: Polyquatemium-7, oder PQ-7), Homopolymeren aus Methacryloylethyltrimethylammonium Chlorid (INCI: Polyquaternium-37, oder PQ-37), quaternären Copolymeren aus Hydroxyethylcellulose und Diallyl-dimethylammoniumchlorid (INCI: Polyquaternium-4, oder PQ-4), polymeren quaternisierten Ammoniumsalzen von Hydroxyethylcellulose, welche mit einem trimethylammonium-substituierten Epoxid modifiziert sind (INCI: Polyquatemium-10, oder PQ-10), depolymerisierten Guar Gum Derivaten, welche quaternisiert sind (INCI: Guar Hydroxypropyl Trimonium Chlorid) oder quaternisierte Guarderivate und quaternären Copolymeren aus Hydroxyethylcellulose und Diallyldemethylammoniumchlorid. In einer bevorzugten Ausfülhrungsform wird das kationische Polymer ausgewählt aus der Gruppe, die gebildet wird von Polyquaternium-7, Polyquatomium-10 und kationischen Guar-Derivaten.

Weiterhin können mit Vorteil kationische Polymeren gemäß der Lehre der EP 1 767 554 A1 Verwendung finden, die seitens der Anmelderin unter der Marke Polyquart Pro vertrieben werden. Die erfindungsgemäßen Mikroemulsionen enthalten vorzugsweise 0,05 bis 2 Gew.-% dieser kationischen Polymere.

Weiterhin sind noch optional weitere Inhaltsstoffe möglich, wie Biozide, Konservierungsmittel, pH-Regulantien, Farbstoffe, Entschäumer, oder Parfüme als Komponente (g). Bevorzugt sind hier pH-Regulantien, insbesondere anorganische oder organische Säuren, wie Citronensäure oder Benzoesäure, sowie Biozide und / oder Konservierungsmittel enthalten.

Diese optionalen Komponenten sind in Summe vorzugsweise in Mengen von 1 bis 30 Gew.-% und insbesondere in Mengen von 2 bis 15 Gew.-% in den Mikroemulsionen enthalten, jeweils bezogen auf das Gesamtgewicht der Mikroemulsion.

In einer weiteren bevorzugten Ausftührungsform wird eine Mikroemulsion verwendet enthaltend (alle Angaben bezogen auf Aktivsubstanz (AS)):
4-25 Gew.-% der Komponente (a)
4 - 12 Gew.% der Komponente (b)
5 - 30 Gew.-% der Komponente (c)
1 - 15 Gew.-% der Komponente (d)
5 - 20 Gew.-% der Komponente (e)
0-10 Gew.-% der Komponente (f)
0-5 gew.-% anderer Inhaltsstoffe (g)
mit der Maßgabe, dass die Summe aus (a) bis (g) 100 ergibt.

Die Mikroemulsionen können frei von der kationischen Komponente (f) formuliert werden. Dann sind die folgenden Zusammensetzungen bevorzugt (bezogen auf AS):
4 - 25 Gew.-% Komponente (a) Alkyl(oligo)glycosid
4 - 12 Gew.-% Komponente (b) Co-Tensid - vorzugsweise ein Glycerinester
5 - 30 Gew.-% Komponente (c) org. Ölphase - vorzugsweise ein Dialkylether
1-15 Gew.-% Komponente (d) Wachs - vorzugsweise ein Alkylester und
0 - 5 Gew.-% weitere Inhaltsstoffe (g)
und der Rest auf 100 Gew.-% ist Wasser.

Vorteilhaft sind auch Mikroemulsionen, die kationische Komponenten f) enthalten (alle Angaben bezogen auf AS). Hier sind die folgenden Mengenbereiche bevorzugt:
4 - 25 Gew.-% Komponente (a) Allcyl(oligo)glycosid
4 - 12 Gew.-% Komponente (b) Co-Tensid - vorzugsweise eine Glycerinester
5 - 30 Gew.-% Komponente (c) org. Ölphase - vorzugsweise ein Dialkylether
1 - 15 Gew.-% Komponente (d) Wachs - vorzugsweise eine Alkylester
0,5 -10 Gew.-% Komponente (f)
0 - 5 Gew.-% andere Inhaltsstoffe (g)
und der Rest auf 100 Gew.-% Wasser.

Die wässerigen Mikroemulsionen gemäß der vorliegenden Beschreibung weisen vorzugsweise eine pH-Wert zwischen 3 und 9, wobei die Bereiche von 4 bis 8 bzw. von 5,5 bis 7,5 und 6,5 bis 7,0 besonders vorteilhaft sein können.

### Herstellung der Mikroemulsionen

Hergestellt werden diese Emulsionen beispielsweise indem man zunächst in einem ersten Schritt eine Mikroemulsion herstellt, enthaltend vorzugsweise mindestens 10 - 20 Gew.-% eines Alkyl(oligo)glycosids der allgemeinen Formel R¹O-[G]ₚ in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht und vorzugsweise 4 - 10 Gew.-% eines Esters von Glycerin mit einer gesättigten oder ungesättigten Fettsäure der Kettenlänge C12-C22 und vorzugsweise 5 - 30 Gew.-% eines Ölkörpers und den Rest auf 100 Gew.-% Wasser zusammengibt und diese Mischung, ggf. unter Erwärmung auf Temperaturen von 30 bis 80 °C verrührt.

In einer bevorzugten Ausführungsform werden die Mikroemulsionen nach der Lehre der WO 08/15507 A1 hergestellt: Das dort offenbarte Verfahren ist ein zweistufiger Prozess, bei dem im ersten Schritt auf an sich bekannte Weise eine Mikroemulsion hergestellt wird. Die Herstellung der Mikroemulsionen in Schritt 1 erfolgt vorzugsweise durch Vermischen der Ölphase mit den weiteren öllöslichen Inhaltsstoffen. Erwärmen der Ölphase über den Schmelzpunkt aller Bestandteile und anschließender Zugabe der wässrigen tensidhaltigen Phase. Die thermodynamisch stabile Mikroemulsion bildet sich dann spontan, ggf. muss noch etwas gerührt werden.

Die Mikroemulsionen werden erfindungsgemäß zu an sich bekannten Reinigungsmitteln hinzu gegeben, um bei diesen die Hautfreundlichkeit zu verbessern. Bevorzugt werden die Mikroemulsionen Handgeschirrspülmitteln zugesetzt.

Dazu ist es vorteilhaft, wenn die Mikroemulsion bei Temperaturen von 35 bis 65 °C, vorzugsweise von 40 bis 50 °C mit den anderen Bestandteilen des Reinigungsmittels vermischt wird. Wegen des Anteils an der wasserunlöslichen Ölphase, bzw. der Wachskomponente kann es bei niedrigeren Verarbeitungstemperaturen zu Trübungen der Emulsion kommen.

Die Mikroemulsionen werden vorzugsweise zu flüssigen Reinigungsmitteln hinzugefügt, da diese sowohl von der Verarbeitung als auch von der Wirkung her von Vorteil ist. Die Reinigungsmittel enthalten typischerweise anionische und/oder amphotheren und/oder nichtionische Tenside sowie Wasser und ggf. weitere für solche Mittel typischen Inhaltsstoffe.

Als Reinigungsmittel werden im Sinne der vorliegenden technische Lehre insbesondere solche Mittel verstanden, die bei der Benutzung direkt mit der menschlichen Haut in Kontakt kommen können. Dazu zählen insbesondere Allzweckreiniger, Bad- oder Kochenreiniger, Boden- und Teppichreiniger und Handgeschirrspülmittel.

Die Herstellung der Reinigungsmittel erfolgt in an Sich bekannter Art und Weise. Die Mikroemulsionen gemäß der obigen Beschreibung werden den Inhaltsstoffen der Reinigungsmittel hinzugefügt, wobei die Wirkung erfindungsgemäß nur dann auftritt wenn die fertige Mikroemulsion mit allen bestimmungsgemäßen Inhaltsstoffen mit den restlichen Inhaltstoffen des Reinigungsmittels zusammen gebracht wird.

Die Zugabe einzelner Bestandteile führt nicht zum gewünschten Ergebnis; Wesentlich ist hier das Merkmal der Mikroemulsion, um die vorliegende Lehre zu verwirklichen.

Es ist hier weiterhin bevorzugt, dass die Zugabe der Mikroemulsion zu den weiteren Bestandteilen des Reinigungsmittels (oder die Zugabe der Bestandteile zu der Mikroemulsion) bei erhöhten Temperaturen, vorzugsweise bei 50 bis 90 °C und vorzugsweise bei 60 bis 80 °C vorgenommen wird, um eine vollständige Durchmischung zu erreichen.

Typische Beispiele für anionische Tenside sind Seifen, Alkylbenzolsulfonate, Alkansulfonate, Olefinsulfonate, Alkylethersulfonate, Glycerinethersulfonate, α -Methylastersulfonate, Sulfofettsäuren, Alkylsulfate, Fettalkoholethersulfate, Glycerinethersulfate, Fettsäureothersulfate, Hydroxymischethersulfate, Monoglycerid-(ether)sulfate, Fettsäureamid(other)sulfate, Mono- und Dialkylsulfosuccinate, Mono-und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäuropolyglycolester, Pettsäureamidpolyglycolether, Fettaminpolyglycolether, alkoxylierte Triglyceride, Mischether bzw. Mischformale, Alk(en)yloligoglycoside, vorzugsweise Alkyl(oligo)glucoside, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide.

Sofern die nichtionischen Tenside Polyglycaletherketten enthalten, können diese eine konventionelle, vorzugsweise jedoch eine eingeengte Homologenverteilung aufweisen. Typische Beispiele für kationische Tenside sind quartäre Ammoniumverbindungen und Esterquats, insbesondere quaternierte Fettsäuretrialkanolaminestersalze.

Typische Beispiele für amphotere bzw. zwitterionische Tenside sind Alkylbetaine, Alkylamidobetaine, Aminopropionate, Aminoglycinate, Imidazoliniumbetaine und Sulfobetaine. Besonders bevorzugt sind anionische Tenside enthalten und hier insbesondere Alkylethersulfate.

Alkylethersulfate ("Ethersulfate") stellen bekannte anionische Tenside dar, die großtechnisch durch SO₃- oder Chlorsulfonsäure (CSA)-Sulfatierung von Fettalkohol-oder Oxoalkoholpolyglycolethem und nachfolgende Neutralisation hergestellt werden. Im Sinne der Erfindung kommen Ethersulfate in Betracht, die der Formel (II) folgen,R²O-(CH₂CH₂O)ₘSO₃X (II) in der R² für einen linearen oder verzweigten Alkyl- und/oder Alkenylrest mit 6 bis 22 Kohlenstoffatomen, n für Zahlen von 1 bis 10 und X für ein Alkali- und/oder Erdalkalimetall, Ammonium, Alkylammonium, Alkanolammonium oder Glucammonium steht. Typische Beispiele sind die Sulfate von Anlagerungsprodukten von durchschnittlich 1 bis 10 und insbesondere 2 bis 5 Mol Ethylenoxid an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen in Form ihrer Natrium- und/oder Magnesiumsalze. Die Ethersulfate können dabei sowohl eine konventionelle als auch eine eingeengte Homologenverteilung aufweisen. Besonders bevorzugt ist der Einsatz von Ethersulfaten auf Basis von Addukten von durchschnittlich 2 bis 3 Mol Ethylenoxid an technische C12/14- bzw. C12/18-Kokosfettalkoholfraktonen in Form ihrer Natrium- und/oder Magnesiumsalze.

Die Reinigungsmittel können weiterhin Farbstoffe, Duftstoffe, Perlglanzmittel, Trübungsmittel, Komplexbildner anorganische oder organische Säuren und/oder Basen, Builder, Bleichmittel, Entschäumer, aber auch Polymere (z.B. als Verdicker aber auch als Builder), Hydrotope bzw. Lösungsvermittler und dergleichen enthalten. Vorzugsweise enthalten die Reinigungsmittel Polymere, wobei zu den Details auf die obige Beschreibung hierzu verwiesen wird. Diese Stoffe werden dann üblicherweise in Mengen von insgesamt bis zu 20 Gew.-% vorzugsweise aber nur bis maximal 15 Gew.-% und insbesondere von 1,5 bis 5 Gew.- % bezogen auf das Gesamtgewicht der Reinigungsmittel eingesetzt.

Der pH-Wert der Reinigungsmittel liegt vorzugsweise im Bereich von 5,0 bis 10,0 vorzugsweise 5,5 bis 8,0. Bevorzugt weisen die Reinigungsmittel einen pH-Wert im Bereich von 6 bis 7,5 auf. Bei sauren Reinigern, wie sie im Badbereich häufig Verwendung finden, sind aber auch deutlich kleinere pH-Wert, typischerweise von 2 bis 5, vorzugsweise von 3,5 bis 4,5 möglich.

Ein weiterer Gegenstand der Anmeldung betrifft daher die Verwendung von Mikroemulsionen nach der obigen Beschreibung zur Verbesserung der sensorischen Eigenschaften von Reinigungsmitteln, insbesondere von wässerigen Handgeschirrspülmitteln.

Unter sensorischen Eigenschaften werden die Eigenschaften von Mitteln verstanden, die zu einer Veränderung der Sinneswahrnehmung durch Menschen führen können, vorliegend ist damit insbesondere das Hautgefühl gemeint, dass durch direkten Kontakt der menschlichen Haut mit einem Stoff oder eine Stoffmischung ausgelöst wird. In der Praxis wird dieses Hautgefühl z.B. durch Panel-Test an Probanden ermittelt, die ihre Sinneseindrücke in Bezug auf bestimmte Parameter, wie "Trockenheit der Haut", "Weichheit der Haut" etc. durch Benotungen qualifizieren. Durch Zusatz der erfindungsgemäßen Mikroemulsionen zu Reinigungsmittel lassen sich diese sensorischen Eindrücke nach Kontakt mit dem jeweiligen Reinigungsmittel der Probanden verbessern.

Die Mikroemulsionen werden vorzugsweise in Mengen von 0,1 bis 20 Gew.-%, vorzugsweise von 0,2 bis 10 Gew.-%, bezogen auf das Gesamtgewicht des Reinigungsmittels eingesetzt, um den gewünschten Erfolg zu erzielen.

Ein letzter Gegenstand betrifft daher ein wässeriges Handgeschirrspülmittel, enthaltend anionische und amphotere Tenside, sowie in Mengen von 0,2 bis 10 gew.- %, bezogen auf das Gesamtgewicht der Handgeschirrspülmittel eine Mikroemulsion gemäß der obigen Beschreibung. Solche Handgeschirrspülmittel können noch weiterhin 5 bis 25 Gew.-% an anionischen Tensiden enthalten, 5 bis 25 Gew.-% an amphoteren Tensiden und 0 bis 1 Gew.-% an kationischen Polymeren (bevorzugt vom Typ des PQ-7 und/oder PQ-10 [nach INCI]), sowie Wasser bis 100 Gew.-%.

### Beispiele

Es wurden die folgenden Mikroemulsionen M1 und M2 hergestellt:

| | **M1** | **M2** |
|---|---|---|
| **Inhaltsstoffe** | **Menge** **[Gew.-%]** | **Menge** **[Gew.-%]** |
| | | |
| Glycerinmonooleat | 8,0 | 8,0 |
| Dioctylether | 25,0 | 25,0 |
| Hexadecylhexadecanoat | 10,0 | 10,0 |
| C₁₂-C₁₆-Fettalkohol-1.4-glucosid | 20,4 | 20,4 |
| C₈-C₁₆-Fettalkohol-1.4-glucosid | 13,6 | 13,6 |
| Trimethylhexadecylammoniumchlorid | - | 5,0 |
| Benzoesäure | 1,0 | 1,0 |
| Zitronensäure | 2,0 | 2,0 |
| Transmission bei 40 °C | 99 % | 99 % |
| Wasser | Rest auf 100 Gew.-% | Rest auf 100 Gew.-% |

Mit den beiden Mikroemulsionen M1 und M2 wurden zwei Handgeschinrspülmittel H1 und H2 hergestellt - Zum Vergleich wurde ein Mittel H3 ohne Mikroemulsion herangezogen - alle Mengenangaben in der Tabelle beziehen sich auf Aktivsubstanz (AS):

| | **H1** | **H2** | **H3** |
|---|---|---|---|
| **Inhaltsstoffe** | **Menge** **[Gew.-%]** | **Menge** **[Gew.-%]** | **Menge** **[Gew.-%]** |
| | | | |
| Alkylethersulfat ¹⁾ | 12,0 | 12,0 | 12,0 |
| Dimethyllcokosacylamidopropyl-ammoniumacetobetain²⁾ | 3,0 | 3,0 | 3,0 |
| Kationisches Polymer | 0,50 | - | - |
| Mikroemulsion M1 | 5,0 | - | - |
| Mikroemulsion M2 | - | 10,0 | - |
| Verdicker ³⁾ | 1,56 | 1,56 | - |
| Biozid⁴⁾ | 0,2 | 0,2 | 0,2 |
| NaCl | 1,0 | 1,0 | - |
| Wasser | Rest auf 100 Gew.-% | Rest auf 100 Gew.-% | Rest auf 100 Gew.-% |

| | | | |
|---|---|---|---|
| ¹⁾ Texapon N 70 (Fa. Cognis) ²⁾ Dehyton K (Fa. Cognis) ³⁾ Arlypon TT (Fa. Cognis) ⁴⁾ Microcare IT (Fa. Thor) | | | |

Die drei Rezepturen H1-H3 wurden in einem Unteramtest durch Testpersonen paarweise geprüft. Dazu wurden den Testpersonen die Mittel in einer Konzentration von 10 g/l auf den angefeuchteten Unterarm getropft und dort 30 Sekunden belassen. Danach wurde der Arm 5 Sekunden lang mit Wasser gespült. Die Testpersonen wurden gebeten ihre Eindrücke für die Bereiche "Verteilung des Mittel", "Weichheit nach Auftragung", "Weichheit nach 30 Sekunden" "Glätte nach Auftragen und nach 30 Sekunden", "Trockenheit nach Auftragung und nach 30 Sekunden" und "Akzeptanz" zu bewerten. Es wurden die Rezepturen H1 und H3 sowie die Rezepturen H2 und H3 gegeneinander geprüft.

Dabei zeigte sich, dass jeweils die Rezeptur H1 gegenüber H3 und die Rezeptur H2 gegenüber H3 bevorzugt wurden.

## Patentansprüche

1. Verwendung von Mikroemulsionen, enthaltend mindestens
(a) ein Alkyl(oligo)glycosid
(b) ein von (a) unterschiedliches Co-Tensid
(c) eine nicht-wasserlösliche organische ölkomponente
(d) ein Wachs
(e) und Wasser
zur Herstellung von Reinigungsmitteln,
wobei als Co-Tensid (b) Polyolester enthalten sind,
und wobei als Wachskomponente (d) Wachse auf Basis von Estern aus Monocarbonsäuren mit mindestens 14 C-Atomen mit Fettalkoholen mit mindestens 14 C-Atomen ausgewählt werden.

2. Verwendung nach Anspruch 1, **dadurch gekennzeichnet, dass** die Mikroemulsion ein Alkyl(oligo)glycosid (a) gemäß der allgemeinen Formel R¹O-[G]ₚ in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht, enthalt.

3. Verwendung nach mindestens einem der Ansprüchen 1 bis 2, **dadurch gekennzeichnet, dass** als Co-Tensid (b) Ester des Glycerins und insbesondere Monoesters der Glycerins enthalten sind.

4. Verwendung nach mindestens einem der Ansprüche 1 bis 3, **dadurch gekennzeichnet, dass** als organische Ölkomponente (c) verzweigte Fettalkohole, C₆-C₂₂-Dialkylether, Ester von verzweigten oder linearen Monocarbonsäuren mit 6 bis 22 C-Atomen mit linearen oder verzweigten Fettalkoholen mit 6 bis 22 C-Atomen, Kohlenwasserstoffe, Silikonöle, oder deren Mischungen enthalten sind.

5. Verwendung nach mindestens einem der Ansprüche 1 bis 4, **dadurch gekennzeichnet, dass** die Mikroemulsion als zusätzliche Komponente (f) eine kationische Verbindung, insbesondere eine quaternäre Ammoniumverbindung und/oder ein kationisches Polymer enthält.

6. Verwendung nach mindestens einem der Ansprüche 1 bis 5, **dadurch gekennzeichnet, dass** die Mikroemulsion noch optional weitere Inhaltsstoffe, wie Biozide, Konservierungsmittel, pH-Regulantien, Farbstoffe oder Parfüme als Komponente (g) enthält.

7. Verwendung von Mikroemulsionen nach mindestens einem der Ansprüche 1 bis 6 zur Verbesserung der Sensorischen Eigenschaften von Reinigungsmitteln, insbesondere von wässerigen Handgeschirrspülmitteln.

8. Wässeriges Handgeschirrspülmittel, enthaltend anionische und amphotere Tenside, sowie in Mengen von 0,2 bis 10 Gew.-%, bezogen auf das Gesamtgewicht der Handgeschirrspülmittel eine Mikroemulsion gemäß der Beschreibung im Anspruch 1.

## Claims

1. Use of microemulsions comprising at least
(a) an alkyl (oligo)glycoside
(b) a cosurfactant different from (a)
(c) a non-water-soluble organic oil component
(d) a wax
(e) and water
for producing cleaning agents,
polyol esters being present as cosurfactant (b), and waxes based on esters of monocarboxylic acids having at least 14 carbon atoms with fatty alcohols having at least 14 carbon atoms being selected as wax component (d).

2. Use according to Claim 1, **characterized in that** the microemulsion comprises an alkyl (oligo)glycoside (a) according to the general formula R¹O-[G]ₚ, in which R¹ is an alkyl and/or alkenyl radical having 4 to 22 carbon atoms, G is a sugar radical having 5 or 6 carbon atoms and p is numbers from 1 to 10.

3. Use according to at least one of Claims 1 to 2, **characterized in that** esters of glycerol and in particular monoesters of glycerol are present as cosurfactant (b).

4. Use according to at least one of Claims 1 to 3, **characterized in that** branched fatty alcohols, C₆-C₂₂ dialkyl ethers, esters of branched or linear monocarboxylic acids having 6 to 22 carbon atoms with linear or branched fatty alcohols having 6 to 22 carbon atoms, hydrocarbons, silicone oils, or mixtures thereof are present as organic oil component (c).

5. Use according to at least one of Claims 1 to 4, **characterized in that** the microemulsion comprises a cationic compound, in particular a quaternary ammonium compound and/or a cationic polymer, as additional component (f).

6. Use according to at least one of Claims 1 to 5, **characterized in that** the microemulsion also optionally comprises further ingredients such as biocides, preservatives, pH regulators, dyes or perfumes as component (g).

7. Use of microemulsions according to at least one of Claims 1 to 6 for improving the sensory properties of cleaning agents, in particular of aqueous hand dishwashing detergents.

8. Aqueous hand dishwashing detergent comprising anionic and amphoteric surfactants, and, in amounts of 0.2 to 10% by weight, based on the total weight of the hand dishwashing detergent, a microemulsion according to the description in Claim 1.

## Revendications

1. Utilisation de microémulsions, contenant au moins
(a) un alkyl(oligo)glycoside
(b) un co-tensioactif différent de (a)
(c) un composant huileux organique non hydrosoluble
(d) une cire
(e) et de l'eau
pour la fabrication de produits de nettoyage,
des polyesters étant contenus en tant que co-tensioactif (b),
et des cires à base d'esters d'acides monocarboxyliques comportant au moins 14 atomes de carbone avec des alcools gras comportant au moins 14 atomes de carbone étant choisies en tant que composant de type cire (d).

2. Utilisation selon la revendication 1, **caractérisée en ce que** la microémulsion contient un alkyl(oligo)glycoside (a) selon la formule générale R¹O-[G]ₚ, dans laquelle R¹ représente un radical alkyle et/ou alcényle ayant de 4 à 22 atomes de carbone, G un radical glucidique ayant 5 ou 6 atomes de carbone et p des nombres de 1 à 10.

3. Utilisation selon au moins l'une quelconque des revendications 1 et 2, **caractérisée en ce que** des esters du glycérol et en particulier des monoesters du glycérol sont contenus en tant que co-tensioactif (b).

4. Utilisation selon au moins l'une quelconque des revendications 1 à 3, **caractérisée en ce que** des alcools gras ramifiés, des éthers dialkyliques en C₆-C₂₂, des esters d'acides monocarboxyliques linéaires ou ramifiés ayant de 6 à 22 atomes de carbone avec des alcools gras linéaires ou ramifiés ayant de 6 à 22 atomes de carbone, des hydrocarbures, des huiles de silicone, ou des mélanges de ceux-ci, sont contenus en tant que composant huileux organiques (c).

5. Utilisation selon au moins l'une quelconque des revendications 1 à 4, **caractérisée en ce que** la microémulsion contient en tant que composant supplémentaire (f) un composé cationique, en particulier un composé d'ammonium quaternaire et/ou un polymère cationique.

6. Utilisation selon au moins l'une quelconque des revendications 1 à 5, **caractérisée en ce que** la microémulsion contient encore, en tant que composant (g), d'autres composants optionnels, tels que des biocides, conservateurs, régulateurs du pH, colorants ou parfums.

7. Utilisation de microémulsions selon au moins l'une quelconque des revendications 1 à 6, pour l'amélioration des propriétés sensorielles de produits de nettoyage, en particulier de produits aqueux pour lavage de la vaisselle à la main.

8. Produit aqueux pour lavage de la vaisselle à la main, contenant des tensioactifs anioniques et des tensioactifs amphotères, ainsi qu'en quantités de 0,2 à 10 % en poids, par rapport au poids total des produits pour lavage de la vaisselle à la main, une microémulsion selon la description dans la revendication 1.
